# EUROPEAN PATENT APPLICATION

(11) **EP 3 006 008 A1**
(43) Date of publication of application: **13.04.2016**
(21) Application number: 14803562.9
(22) Date of filing: 30.05.2014
(51) Int. Cl.: A61J 1/16, A61M 5/14

(54) **IV STAND, AND ATTACHMENT FOR IV STAND**

(30) Priority: 31.05.2013 JP 2013116002; 31.05.2013 JP 2013116003
(71) Applicant: Okamura Corporation, Kanagawa 220-0004 (JP)
(72) Inventor: YAMAMOTO, Noriko, Sabae-shi Fukui 916-0027 (JP); MURATA, Chiaki, Toyonaka-shi Osaka 560-0053 (JP); SAKAKIBARA, Yoshihiro, Yokohama-shi Kanagawa 220-0004 (JP); ONO, Naho, Yokohama-shi Kanagawa 220-0004 (JP)
(74) Representative: Cabinet Laurent & Charras
(86) International application number: PCT/JP2014/064447
(87) International publication number: WO 2014/192926

(57) **Abstract**

The IV stand includes: a tray having an object placing surface on which an object is capable of being placed; and a guide member that is arranged above at least a part of the object placing surface of the tray and prevents the object placed on the tray from falling down, in which the tray and the guide member are directly or indirectly coupled to the support pole unit, and a holding space that allows an operator to hold the guide member as a holding handle is secured between the guide member and the tray. With the aforementioned structure, it is possible to provide an IV stand that makes it possible to enhance the usability and operability without deteriorating appearance and increasing occupied space.

## Description

### Technical Field

The present invention relates to an IV stand and an attachment for an IV stand that are used when an IV is administered to a patient or on other occasions in a hospital, a nursing home, a house of the patient, or other places.

Priority is claimed on Japanese Patent Application No. 2013-116002, filed on May 31, 2013, and on Japanese Patent Application No. 2013-116003, filed on May 31, 2013, the contents of which are incorporated herein by reference.

### Background Art

In a hospital, a nursing home, the house of a patient, or other places, an IV is often administered to a patient as a therapeutic activity. An IV is a mechanism in which an infusion pack is arranged above a patient and the gravity is utilized to infuse the infusion solution in the infusion pack into the body of the patient. Therefore, it is a general practice to use an IV stand that supports the infusion pack above the patient.

In general, the basic structure of an IV stand includes: a leg unit that is placed on a floor surface ; a support pole unit extending along the vertical direction and attached to the leg unit; and an infusion solution hanger attached to an upper end of the support pole unit (for example, see Patent Documents 1 to 3).

The IV stands described in Patent Documents 1 to 3 has a structure in which an infusion solution hanger includes: a pair of arm units that extend substantially horizontally from an upper end of the support pole unit; and hook units each of which is provided at a front end of each arm unit for directly hanging an infusion pack. Furthermore, the IV stands have a leg unit provided with casters so that a patient, nurse, or someone else can freely move the stand as required.

It is desirable that, for better usability, the IV stand be provided with a tray (an object placement table) which is used as a surface on which belonging(s) of a patient are placed when the patient is moving or an operation surface that is used when the operation of hanging infusion pack(s) is carried out. With this structure, it is possible to further enhance the usability of the IV stand.

Furthermore, for the IV stand to be moved, it is desirable that a holding handle be provided that is formed integrally with the support pole unit of the IV stand. IV stands provided with this holding handle are conventionally devised (for example, see Patent Document 2).

The IV stand described in Patent Document 2 is provided with a holding handle made of: an annular handle main unit; a boss section that is fixed to an outer surface of the support pole unit; and a coupling unit that couples the boss section to the handle main unit. In the IV stand described in Patent Document 2, it is possible to move the stand while the holding handle is held from any direction around the support pole unit. Furthermore, it is possible to operate the stand while a plurality of persons hold the holding handle. Therefore, the operability of the IV stand is favorable.

### Citation List

### Patent Documents

Patent Document 1: Japanese Unexamined Patent Application, First Publication No. 2012-010718
Patent Document 2: Japanese Unexamined Patent Application, First Publication No. 2013-017653
Patent Document 3: Japanese Patent No. 3466180

### Summary of Invention

### Technical Problem

To enhance the usability and operability of the IV stand, the support pole unit of the IV stand may be provided with the tray and the holding handle as described above. However, if the tray and the holding handle are simply provided on the support pole unit separately at positions spaced away from each other, a plurality of outwardly-extending sections from the support pole unit are formed at the spaced positions on the support pole unit, leading to a possible deterioration in appearance.

Furthermore, it is desirable that, on or above the tray on which an object is placed, a restriction unit that prevents the placed object from falling down be provided. Accordingly, even in the case where the tray and the holding handle are provided on the support pole unit separately at positions spaced away from each other as described above, the tray may be provided with a restriction unit. However, if a tray with a restriction unit is adopted in these cases, it will result not only in a deterioration in appearance as described above but also in an increase in space occupied by the tray around the support pole unit.

Furthermore, if the tray and the holding handle are provided on the support pole unit separately at positions spaced away from each other, attaching them will be difficult.

Therefore, it is an object of the present invention to provide an IV stand that is capable of enhancing the usability and operability without a deterioration in appearance or an increase in occupied space.

Furthermore, it is an object of the present invention to provide an IV stand and an attachment for an IV stand that are capable of enhancing usability and operability without deteriorating appearance or causing difficulties with attachment.

### Solution to Problem

To solve the above problems, the present invention adopts the following.

According to a first aspect of the present invention, there is provided an IV stand in which an infusion pack is hung on a support pole unit, which extends in a vertical direction, via an infusion solution hanger, the IV stand including: a tray having an object placing surface on which an object is capable of being placed; and a guide member that is arranged above at least a part of the object placing surface of the tray and prevents the object placed on the tray from falling down, in which the tray and the guide member are directly or indirectly coupled to the support pole unit, and a holding space that allows an operator to hold the guide member as a holding handle is secured between the guide member and the tray.

As a result, when an object is placed on the object placing surface of the tray, the guide member prevents the object from falling down. Furthermore, by holding the guide member above the tray as a holding handle, an operator such as a patient or a nurse is capable of operating the IV stand. Furthermore, the tray and the guide member are prevented from being spaced widely apart in the vertical direction of the support pole unit.

According to a second aspect of the present invention, in the IV stand according to the first aspect, the tray is provided with a through-hole through which the support pole unit is inserted in the vertical direction. In this case, the support pole unit is not arranged outside the tray. Therefore, it is possible to maintain a small amount of outward extension of the tray from the support pole unit.

According to a third aspect of the present invention, in the IV stand according to the second aspect, the through-hole is provided at a position that is offset from a center of the tray when seen in a top view. In this case, it is possible to secure, on the tray, adequate space for placing an object that does not interfere with the support pole unit.

According to a fourth aspect of the present invention, in the IV stand according to the first to third aspects, the guide member is disposed above the tray so as to be along an outer circumferential edge of the tray.

In this case, it is possible to utilize substantially the whole area of the upper surface of the tray as the object placing surface.

According to a fifth aspect of the present invention, in the IV stand according to the fourth aspect, an inner circumferential surface of the guide member is formed at a position substantially the same as or further outward than an outer circumferential surface of the tray when seen in a top view. In this case, the guide member is not positioned inside the tray when seen in a planar view. Therefore, it is possible to effectively utilize substantially the whole area of the upper surface of the tray as the object placing surface. Furthermore, when the operator holds the guide member as the holding handle, the tray is not likely to be an obstacle.

According to a sixth of the present invention, in the IV stand according to the first to fifth aspects, the guide member is formed in a similar shape to an outer edge of the tray when seen in a top view. In this case, the balance in external appearance is favorable, and the appearance from the outside is not deteriorated.

According to a seventh aspect of the present invention, in the IV stand according to the first to sixth aspects, the guide member is formed in an annular shape when seen in a top view. In this case, when holding the guide member as the holding handle, the operator is capable of holding the guide member from any direction.

According to an eighth aspect of the present invention, there is provided an IV stand in which an infusion pack is hung on a support pole unit, which extends in a vertical direction, via an infusion solution hanger, the IV stand including an attachment which includes: a tray having an object placing surface on which an object is capable of being placed; a holding handle that is spaced away from the tray so as to secure a holding space that allows an operator to hold the holding handle; a coupling unit that couples the tray to the holding handle; and a fixing unit that fixes at least one of the tray and the holding handle to the support pole unit.

As a result, it is possible to place an object on the object placing surface of the tray, and the operator such as a patient or a nurse is capable of operating the IV stand while holding the holding handle. Furthermore, it is possible to attach the tray and the holding handle as an integrated attachment to the support pole unit via the fixing unit.

According to a ninth aspect of the present invention, in the IV stand according to the eighth aspect, the fixing unit is provided in the coupling unit. In this case, it is possible to make the whole of the attachment more compact in size, and thereby make the external appearance favorable.

According to a tenth aspect of the present invention, in the IV stand according to the eighth or ninth aspect, the tray is provided with a through-hole through which the support pole unit is inserted in the vertical direction. In this case, the support pole unit is not arranged outside the tray. Therefore, it is possible to maintain a small outward extension of the tray from the support pole unit.

According to an eleventh aspect of the present invention, in the IV stand according to the eighth to tenth aspects, the holding handle is formed in a similar shape to an outer edge of the tray when seen in a top view. In this case, the balance in external appearance is favorable, and the appearance from the outside is not deteriorated.

According to a twelfth aspect of the present invention, in the IV stand according to the eighth to eleventh aspect, the holding handle is formed in an annular shape when seen in a top view. In this case, when holding the holding handle, the operator is capable of holding the holding handle from any direction.

According to a thirteenth aspect of the present invention, there is provided an attachment for an IV stand that is attached to a support pole unit extending in a vertical direction, the attachment including: a tray having an object placing surface on which an object is capable of being placed; a holding handle that is spaced away from the tray so as to secure a holding space that allows an operator to hold the holding handle; a coupling unit that couples the tray to the holding handle; and a fixing unit that fixes at least one of the tray and the holding handle to the support pole unit.

### Advantageous Effects of Invention

According to the present invention, it is possible to allow the guide member that is disposed above the tray by a predetermined distance to function both as a restriction unit for preventing an object from falling down and a holding handle. Therefore, without a deterioration in appearance or an increase in occupied space that are caused by the tray and the holding handle being separated from each other on the support shaft, it is possible to place an object on the tray with ease, and to allow the operator to operate the IV stand with ease by holding the guide member.

Furthermore, according to the present invention, it is possible to attach the tray and the holding handle as an integrated attachment to the support pole unit in a compact manner. Therefore, without deteriorating appearance and causing difficulties with attachment, it is possible to place an object on the tray with ease, and to allow the operator to operate the IV stand with ease by holding the holding handle.

### Brief Description of Drawings

FIG. 1 is a perspective view of an IV stand according to a first embodiment of the present invention.
FIG. 2 is a perspective view of the IV stand according to the first embodiment of the present invention.
FIG. 3 is a perspective view of a portion of the IV stand according to the first embodiment of the present invention.
FIG. 4 is a perspective view of the portion of the IV stand according to the first embodiment of the present invention.
FIG. 5 is a plan view in which a support pole unit of the IV stand according to the first embodiment of the present invention is cut away.
FIG. 6 is a partial cross-sectional side view of the IV stand according to the first embodiment of the present invention.
FIG. 7 is a top view showing an IV stand according to a modification of the first embodiment of the present invention.
FIG. 8A is a perspective view of a portion of an IV stand according to a second embodiment of the present invention.
FIG. 8B is a plan view in which a support pole unit of the IV stand according to the second embodiment of the present invention is cut away.
FIG. 8C is a plan view in which a support pole unit of an IV stand according to a modification of the second embodiment of the present invention is cut away.
FIG. 9A is a plan view in which a support pole unit of an IV stand according to a third embodiment of the present invention is cut away.
FIG. 9B a plan view in which a support pole unit of an IV stand according to a modification of the third embodiment of the present invention is cut away.
FIG. 10A is a plan view in which a support pole unit of an IV stand according to a fourth embodiment of the present invention is cut away.
FIG. 10B is a plan view in which a support pole unit of an IV stand according to a modification of the fourth embodiment of the present invention is cut away.
FIG. 11 is a plan view in which a support pole unit of an IV stand according to a fifth embodiment of the present invention is cut away.
FIG. 12 is a perspective view of an IV stand according to a sixth embodiment of the present invention,
FIG. 13 is a perspective view of the IV stand according to the sixth embodiment of the present invention.
FIG. 14 is a perspective view of a portion of the IV stand according to the sixth embodiment of the present invention.
FIG. 15 is a perspective view of the portion of the IV stand according to the sixth embodiment of the present invention.
FIG. 16 is a plan view in which a support pole unit of the IV stand according to the sixth embodiment of the present invention is cut away.
FIG. 17 is a partial cross-sectional side view of the IV stand according to the sixth embodiment of the present invention.
FIG. 18 is a top view showing an IV stand according to a modification of the sixth embodiment of the present invention.
FIG. 19A is a perspective view of a portion of an IV stand according to a seventh embodiment of the present invention.
FIG. 19B is a plan view in which a support pole unit of the IV stand according to the seventh embodiment of the present invention is cut away.
FIG. 19C is a plan view in which a support pole unit of an IV stand according to a modification of the seventh embodiment of the present invention is cut away.
FIG. 20A is a plan view in which a support pole unit of an IV stand according to an eighth embodiment of the present invention is cut away.
FIG. 20B a plan view in which a support pole unit of an IV stand according to a modification of the eighth embodiment of the present invention is cut away.
FIG. 21A is a plan view in which a support pole unit of an IV stand according to a ninth embodiment of the present invention is cut away.
FIG. 21B is a plan view in which a support pole unit of an IV stand according to a modification of the ninth embodiment of the present invention is cut away.
FIG. 22 is a plan view in which a support pole unit of an IV stand according to a tenth embodiment of the present invention is cut away.
FIG. 23 is a perspective view of a portion of an IV stand according to an eleventh embodiment of the present invention.

### Description of Embodiments

### (First embodiment)

Hereunder is a description of embodiments of the present invention, with reference to the drawings.

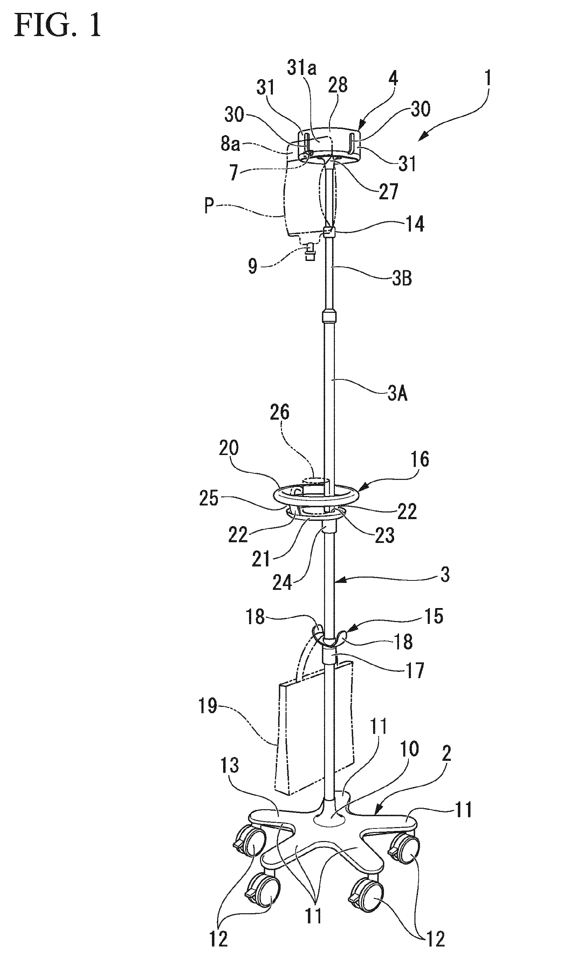

Firstly, a first embodiment shown in FIG. 1 to FIG. 6 will be described. In the embodiments and modifications described below, the same parts are denoted with the same reference symbols, and will not be repetitiously explained.

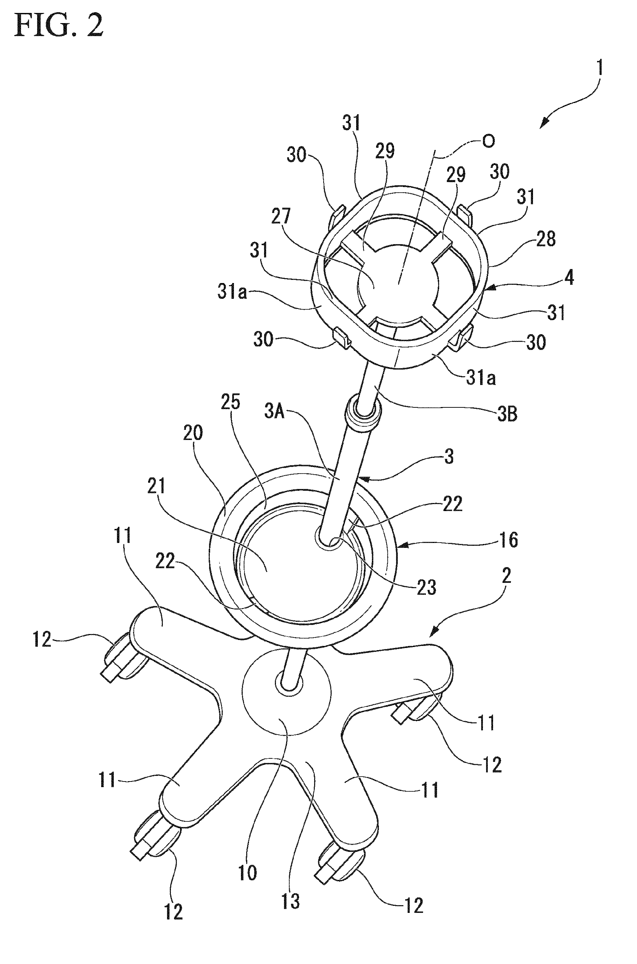

FIG. 1 and FIG. 2 are perspective views showing external appearances of an IV stand 1 according to this embodiment.

The IV stand 1 includes: a leg unit block 2 that is placed on a floor surface of a hospital, a nursing home, a home of a patient, or the like; a support pole unit 3 that is supported on the leg unit block 2 and extends in the vertical direction; and an infusion solution hanger 4 that is attached to an upper end of the support pole unit 3 and hangingly supports an infusion pack P.

The leg unit block 2 includes: five leg frames 11 extending radially toward an outer circumferential side of a boss section 10 which sustains a lower end of the support pole unit 3; and casters 12, each of which is attached to an extension end of each leg frame 11. Therefore, the leg unit block 2 of this embodiment is movable on the floor surface via the casters 12. In the case of this embodiment, an upper side of the boss section 10 and the leg frames 11 is actually covered with a cover member 13 made of resin. However, in FIG. 1 and FIG. 2, the sections on the cover member 13 that correspond to the boss section 10 and the leg frames 11 are denoted with reference symbols 10, 11, for convenience of illustration.

In the case of this embodiment, the support pole unit 3 includes: a hollow outer pole 3A having a lower end which is attached to the leg unit block 2; and a hollow inner pole 3B having an upper end to which the infusion solution hanger 4 is attached. The lower section of the inner pole 3B is slidably inserted into the upper portion of the outer pole 3A. The inside of the support pole unit 3 is equipped with a lock mechanism (not shown in the figures) for fixing the relative positional relationship between the outer pole 3A and the inner pole 3B. With the lock mechanism released, it is possible to appropriately adjust the relative positional relationship between the outer pole 3A and the inner pole 3B (a telescopic length of the support pole unit 3).

In a substantially middle position of the inner pole 3B in the vertical direction, there is installed a release operation sleeve 14 that is used for releasing the lock mechanism. Inside the inner pole 3B, the release operation sleeve 14 is connected to a lock release piece of the lock mechanism. When slid upwardly by an operator such as a nurse, the release operation sleeve 14 releases the lock mechanism. When slid back downwardly, the release operation sleeve 14 maintains the lock mechanism in the locked state again.

Furthermore, in this embodiment, to the outer pole 3A, a hanger hook attachment 15 and a handle attachment 16 (an attachment for the IV stand) are attached so as to be capable of being risen and lowered in an adjustable manner.

The hanger hook attachment 15 includes: a boss section 17 capable of being fastened and fixed to an outer circumferential surface of the outer pole 3A; and a pair of hook units 18, 18 that extend from the boss section 17 in opposite directions. It is possible to appropriately hang an urobag, belongings 19, or the like on each of the hook units 18. The handle attachment 16 will be described in detail later.

The infusion solution hanger 4 includes: a boss section 27 that is fastened and fixed to an upper end of the inner pole 3B of the support pole unit 3; a circumferential wall 28 with a substantially square cylindrical shape that is erected along the vertical direction so as to surround an axis o of the support pole unit 3; coupling poles 29 that couple the boss section 27 to the circumferential wall 28; and four hanger support pieces 30 that protrude from an outside surface of a lower end of the circumferential wall 28 to the outside (the side opposite to the side on which the axis o of the support pole unit 3 is located).

The circumferential wall 28 is formed of four support walls 31 with both ends thereof in the left-right direction curved in an arc shape, in which the support walls 31 are arranged in a square shape so that adjacent support walls 31, 31 are coupled to each other. In the case of this embodiment, the whole of the circumferential wall 28 is integrally formed from a rigid resin material into a square shape. An outer surface 31 a of each support wall 31 (a surface on the side opposite to the side on which the axis o of the support pole unit 3 is located) is formed in a substantially plain shape centering around the central region thereof in the width direction.

The number of the coupling poles 29 is four, each of which is provided so as to couple a lower edge of the corresponding support wall 31 at a central position in the width direction to the outer surface of the boss section 27. The hanger support piece 30 is made of a metal plate member that is formed in a substantially L-shape in a cross-sectional view. A first edge of the L-shape is attached to the lower edge of the corresponding support wall 31 at a central position in the width direction while a second edge of the L-shape extends in the upward direction. A front end of the first edge of the L-shape of the hanger support piece 30 is inserted between the corresponding support wall 31 and the corresponding coupling pole 29, and is fixed to the coupling pole 29. With each hanger support piece 30, a hanging hole 7 of the infusion pack P is engageable, as shown in FIG. 1. Reference symbol 8a in FIG. 1 denotes an upper sealed section of the infusion pack P in which the hanging hole 7 is formed, and reference symbol 9 denotes an outlet pipe of the infusion pack P to which an infusion tube (not shown in the figure) is connected.

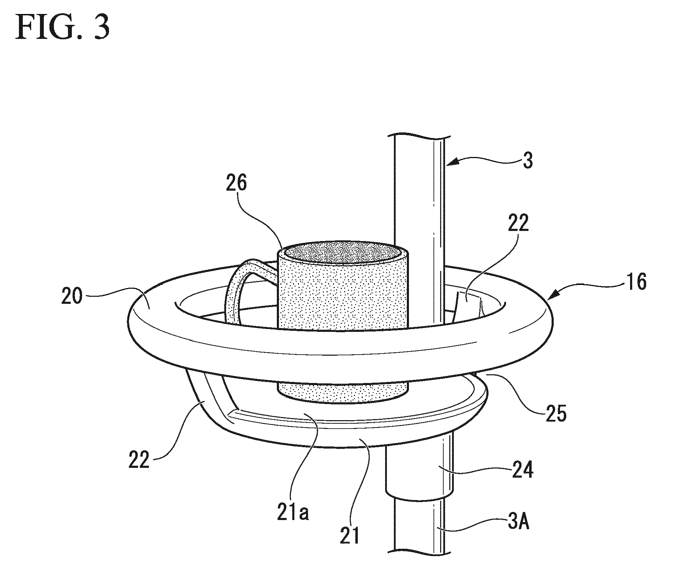

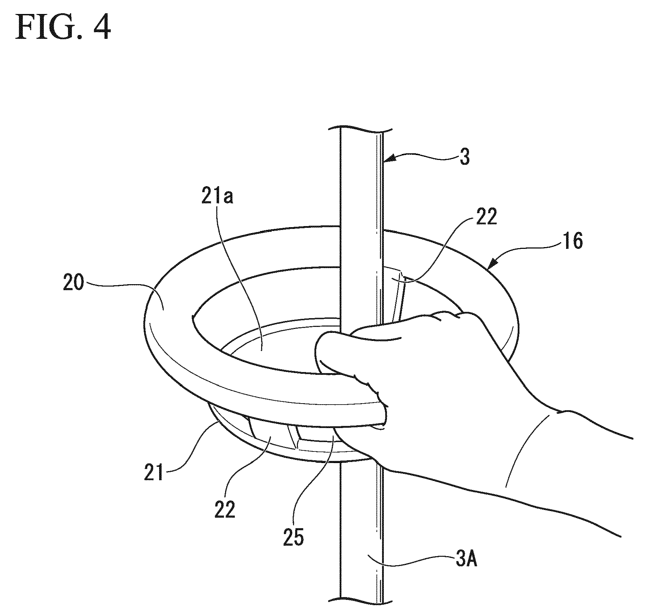

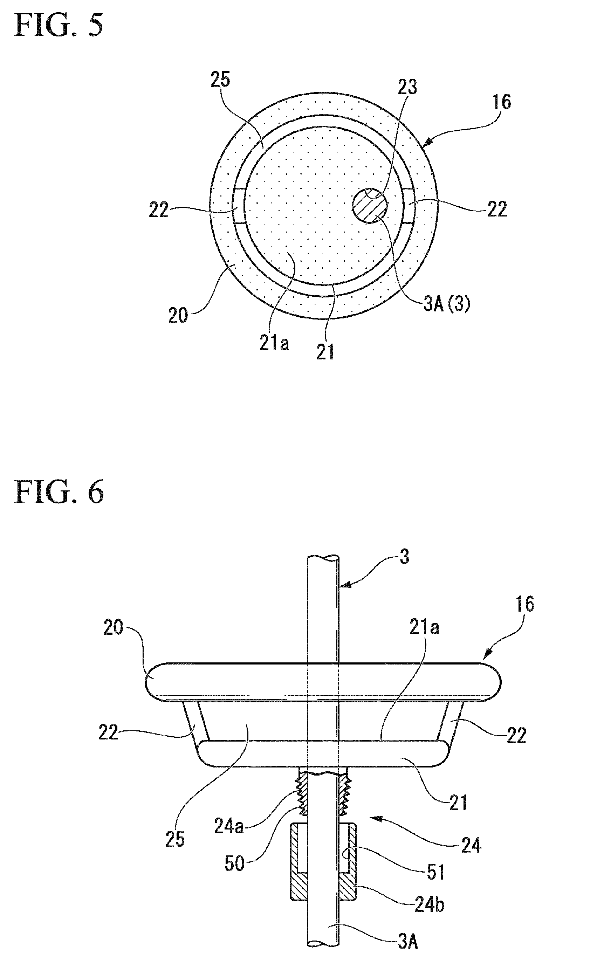

FIG. 3 and FIG. 4 are perspective views showing a portion of the handle attachment 16. FIG. 5 is a top view of a portion of the handle attachment 16 shown with the support pole unit 3 cut away. FIG. 6 is a side view of the portion of the handle attachment 16, which is shown partially in cross-section.

The handle attachment 16 includes: a tray 21 having an upper surface on which an object 26 including an everyday item such as a cup or the like is capable of being placed; a handle main unit 20 (a guide member), which is an annular holding handle arranged above the tray 21 so as to be spaced a predetermined distance away from the tray 21; a pair of coupling poles 22, 22 (coupling units) that couple the tray 21 to the handle main unit 20; and a boss section 24 (a fixing unit) that is fastenable and fixable to the outer circumferential surface of the outer pole 3A.

The tray 21 is formed in a circular flat plate that is a size smaller than an inner circumferential surface of the handle main unit 20. An upper surface of the tray 21 is an object placing surface 21a on which the object 26 is placed. The tray 21 is arranged below the handle main unit 20 so as to be coaxial with the handle main unit 20, and is coupled to the handle main unit 20 by the coupling poles 22, 22 at positions opposite to each other on the outer circumference of the tray 21. The coupling poles 22 are arranged in an inclined manner so as to couple the upper surface of the tray 21 on the outer circumferential side to the lower surface of the handle main unit 20 on the inner circumferential side. The tray 21 is provided with a through-hole 23 that penetrates vertically therethrough at a position that is offset from the central portion thereof. Through the through-hole 23, the outer pole 3A is inserted.

In the case of this embodiment, the tray 21, the coupling poles 22, 22, and the handle main unit 20 are formed from a rigid resin so as to be integral with a part of the boss section 24.

Furthermore, the boss section 24 is provided at a position so as to be coaxial with the through-hole 23 on the lower surface of the tray 21. The boss section 24 is made of: an inner cylinder 24a, with a cylindrical shape divided into half, that is provided so as to protrude from the lower surface of the tray 21; and an outer cylinder 24b that is fitted onto an outside of the inner cylinder 24a. The inner cylinder 24a is deformable inwardly in the radial direction about the divided section, and is provided with an external thread 50 in its outer circumferential surface. The outer cylinder 24b is provided with an internal thread 51 that is threadedly engageable with the external thread 50 of the inner cylinder 24a. With the internal thread 51 being screwed onto the external thread 50 of the inner cylinder 24a, it is possible to press-fix the inner cylinder 24a onto the outer circumferential surface of the support pole unit 3 (the outer pole 3A). Therefore, with the screwing of the outer cylinder 24b of the boss section 24 being released, the handle attachment 16 is movable on the support pole unit 3 (the outer pole 3A). With the outer cylinder 24b being screwed again at a desired position, it is possible to fix the handle attachment 16 to the support pole unit 3 (outer pole 3A).

In the case of this embodiment, the tray 21 is coupled directly to the support pole unit 3 via the boss section 24 while the handle main unit 20 (the guide member) is coupled indirectly to the support pole unit 3 via the tray 21.

Here, below the handle main unit 20 coupled to the tray 21 by the pair of coupling poles 22, 22, a holding space 25 is secured that is wide enough for an operator to hold the handle main unit 20. Therefore, unless the caster 12 of the leg unit block 2 is locked, an operator such as a patient or a nurse is capable of moving the IV stand 1 freely in any direction while holding the handle main unit 20.

Furthermore, although the holding space 25 is secured between the handle main unit 20 and the tray 21, the height from the upper surface of the tray 21 to the handle main unit 20 is set to be lower than that of the object 26 that a patient uses in his or her daily life such as a cup. Therefore, the handle main unit 20 functions also as a guide member (a restriction unit) that restricts the object 26 placed on the tray 21 from falling down. In the case of this embodiment, the through-hole 23 in the tray 21 through which the outer pole 3A is inserted is arranged at a position that is offset from the center of the tray 21. Therefore, on the tray 21, enough object arrangement space is secured that does not interfere with the outer pole 3A.

Furthermore, in this embodiment, the whole area of the upper surface of the handle main unit 20 is formed so as to be substantially horizontal. Therefore, as required, it is also possible to place a large object such as an IV pack P on the upper surface of the handle main unit 20 as well.

As described above, in the IV stand 1 according to this embodiment, the handle attachment 16 to be attached to the support pole unit 3 is provided with: the planar tray 21 with the object placing surface 21a; and the handle main unit 20, which is an annular guide member that is arranged so as to surround the area above the object placing surface 21 a of the tray 21. Moreover, the holding space 25 is secured between the handle main unit 20 and the tray 21. Therefore, it is possible to combine the placement function portion for the object 26 and the holding handle function portion in a compact manner and to install them around the support pole unit 3. As a result, in the IV stand 1 of this embodiment, it is possible to achieve both of the placement of the object 26 around the support pole unit 3 and the secure holding of the IV stand 1 by the operator in moving or on other occasions, without a deterioration in appearance around the support pole unit 3 and an increase in occupied space for placing object.

Furthermore, especially in the IV stand 1, the handle main unit 20 functions also as a guide member (a restriction unit) that restricts the object 26 placed on the object placing surface 21a of the tray 21 from falling down. Therefore, it is possible to further enhance the usability when the object 26 is placed on the tray 21. Therefore, in the case of the IV stand 1, with the handle attachment 16 being provided with the function of limiting the object 26 from falling down, it is possible to limit an increase in space occupied by the handle attachment 16 and to make the appearance of the whole unit favorable.

Furthermore, there may be a case of arranging the tray 21 of the handle attachment 16 outside the support pole unit 3 by using a coupling arm or the like that extends from a side of the tray 21. On the other hand, in the IV stand 1 of this embodiment, it is possible to make smaller an amount of outward extension of the tray 21 from the support pole unit 3 because the tray 21 of the handle attachment 16 is provided with the through-hole 23 and the support pole unit 3 is inserted through the through-hole 23. Therefore, in the IV stand 1, it is possible to further enhance the usability and appearance as an IV stand.

Furthermore, in the IV stand 1 of this embodiment, the through-hole 23 is provided at a position that is offset from the central position of the tray 21 when seen in top view. Consequently, it is possible to secure, on the tray 21, adequate space for placing an object that does not interfere with the support pole unit 3. Therefore, in the IV stand 1, it is possible to inhibit the amount of outward extension of the tray 21 while maintaining the easiness with which the object 26 is placed on the tray 21.

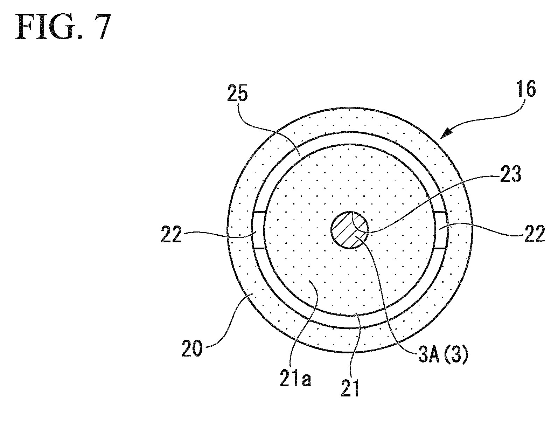

However, in the handle attachment 16, the through-hole 23 is not necessarily required to be provided at a position that is offset from the central position of the tray 21. As is the case with a modification shown in FIG. 7, the through-hole 23 may be provided at the central position of the tray 21.

Furthermore, so long as the handle main unit 20 of the handle attachment 16 is arranged above at least a part of the object placing surface 21a of the tray 21, the handle main unit 20 may not necessarily be arranged above the tray 21 so as to be along the outer circumferential edge of the tray 21. However, in the IV stand 1 of this embodiment, the handle main unit 20 is arranged above the tray 21 so as to be along the outer circumferential edge of the tray 21. Therefore, it is possible to effectively utilize a wide area on the tray 21 as the object placing surface 21a.

Especially in the case of this embodiment, the handle main unit 20 of the handle attachment 16 is arranged so that the inner circumferential surface of the handle main unit 20 is outer than the outer circumferential surface of the tray 21 when seen in a top view. Therefore, it is possible to effectively utilize substantially the whole area of the upper surface of the tray 21 as the object placing surface 21a. Furthermore, this offers an advantage in that, when the operator holds the handle main unit 20 as a holding handle, the tray 21 is unlikely to be an obstacle.

Note that the handle main unit 20 may be formed so that the inner circumferential surface of the handle main unit 20 and the outer circumferential surface of the tray 21 are in substantially the same position when seen in top view.

Furthermore, in the IV stand 1 of this embodiment, the handle main unit 20 of the handle attachment 16 that is arranged above the tray 21 is formed so as to have a similar shape to that of an outer edge of the tray 21. Therefore, the balance in external appearance is favorable, and the appearance from the outside is not deteriorated.

Furthermore, so long as the handle main unit 20 of the handle attachment 16 is arranged above at least a part of the object placing surface 21a of the tray 21, the handle main unit 20 is not necessarily required to have an annular shape. However, in the IV stand 1 of this embodiment, the handle main unit 20 is formed in an annular shape. Therefore, when holding the handle main unit 20 as a holding handle, the operator is capable of holding the handle main unit 20 from any direction in the circumferential area. This produces an advantage of favorable operability.

### (Second embodiment)

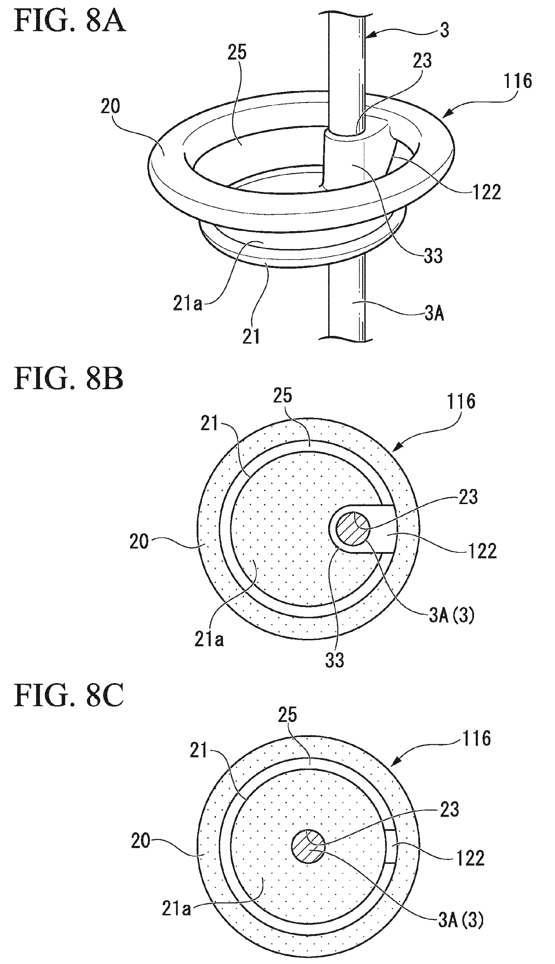

Subsequently, other embodiments shown in FIG. 8A to FIG. 11 will be described. The embodiments shown in FIG. 8A to FIG. 11 are different from the first embodiment only in the structure of the handle attachment, and otherwise have similar structures.

FIGS. 8A and 8B are a perspective view and a top view of a portion of a handle attachment 116 of a second embodiment.

The handle attachment 116 of this embodiment includes: a disc-shaped tray 21 whose upper surface is an object placing surface 21a; and an annular handle main unit 20 (a guide member) that is arranged above the tray 21 so as to be spaced a predetermined distance away from the tray 21. The handle main unit 20 is arranged so as to be coaxial with the tray 21, and a holding space 25 is secured between the handle main unit 20 and the tray 21. Also in the case of this embodiment, the inner circumferential surface of the handle main unit 20 is formed in a circular shape so as to be a size larger than the outer circumferential surface of the tray 21. In the first embodiment, the handle main unit 20 and the tray 21 are coupled by a pair of coupling poles that are arranged at opposite positions on the circumference. However, in this embodiment, the handle main unit 20 and the tray 21 are coupled by a single coupling pole 122.

On the coupling pole 122, a swelling portion 33 with a trapezoidal shape is integrally formed that swells inwardly in the radial direction from the inner circumferential surface of the handle main unit 20 to the outer circumferential edge of the tray 21. In the swelling portion 33 and in a site of the tray 21 that is coupled to the swelling portion 33, there is formed a through-hole 23 through which the outer pole 3A of the support pole unit 3 is inserted in the vertical direction. The swelling portion 33 constitutes at least a part of a fixing unit which fixes the handle attachment 116 to the support pole unit 3. In the case of this embodiment, the through-hole 23 is arranged at a position that is offset from a central position of the tray 21. Note that a fixation device for press-fixing the handle attachment 116 to the support pole unit 3 may be provided in the swelling portion 33, or may be provided on the lower surface side of the tray 21 similarly to the case of the first embodiment.

With the IV stand of this embodiment, it is possible to obtain a basic advantageous effect similar to that of the first embodiment. Furthermore, the handle main unit 20 of the handle attachment 116 is coupled by the coupling pole 122 only at a single location in the circumferential direction. Therefore, when an operator holds the handle main unit 20 as a holding handle, it is possible to expand the area in which the operator can hold the handle main unit 20 without interfering with the coupling pole 122, to thereby enhance the operability. Furthermore, it is possible to further enhance the quality in external appearance.

Furthermore, in the case of this embodiment, the coupling pole 122 that couples the handle main unit 20 to the tray 21 is provided with the swelling portion 33 constituting at least a part of the fixing unit which fixes the handle attachment 116 to the support pole unit 3. Therefore, it is possible to make the handle attachment 116 itself compact in size.

Here, a case where the through-hole 23 is arranged at a position that is offset from the central position of the tray 21 has been described. However, as is the case with a modification shown in FIG. 8C, the through-hole 23 may be arranged at the central position of the tray 21. In the case of this modification, the coupling pole 122 is not provided with a swelling portion, and the tray 21 and the handle main unit 20 are coupled by the coupling pole 122 in a slanting manner.

### (Third embodiment)

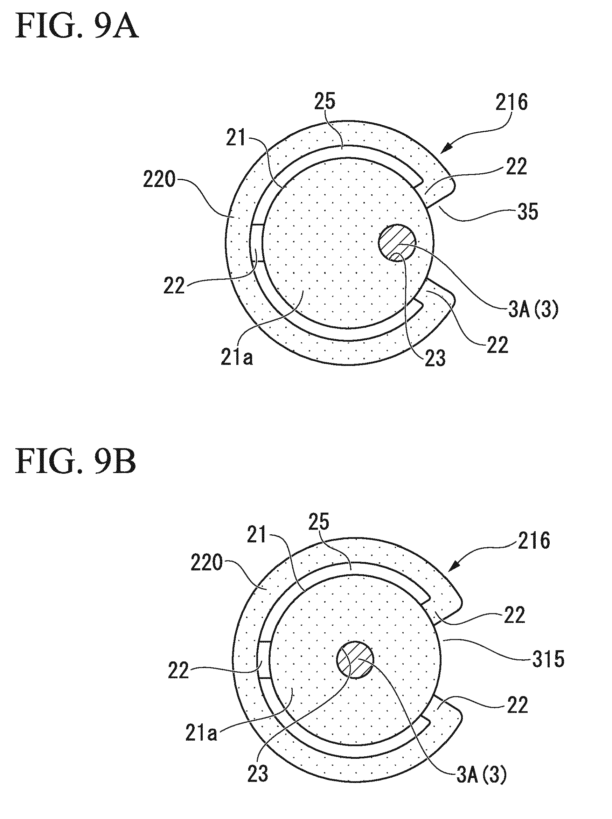

FIG. 9A is a top view of a portion of a handle attachment 216 according to a third embodiment.

The handle attachment 216 of this embodiment includes: a disc-shaped tray 21 whose upper surface is an object placing surface 21a; and a handle main unit 220 (a guide member) that is arranged above the tray 21 so as to be spaced a predetermined distance away from the tray 21. Moreover, a holding space 25 is secured between the handle main unit 220 and the tray 21. However, in the case of this embodiment, the handle main unit 220 does not have a perfect annular shape, but has an arc shape formed by cutting away a part from a circular shape. The handle main unit 220 is arranged so as to be coaxial with the tray 21. The handle main unit 220 is coupled to an outer circumferential edge of the tray 21 by three coupling poles 22 at arc ends on both sides of the handle main unit 220 and at a central portion of the arc. Furthermore, at a position that is offset from the central position of the tray 21, there is provided a through-hole 23 through which a support pole unit 3 is inserted. The inner surface of the handle main unit 220 is formed in a circular shape that is a size larger than the outer circumferential surface of the tray 21.

Furthermore, between the arc ends on both sides of the handle main unit 220, there is provided a recessed space 35 that is recessed in a substantially delta-shape toward the outer circumferential surface of the tray 21 when seen in a top view.

With the IV stand of this embodiment, it is possible to obtain a basic advantageous effect similar to that of the first embodiment. Furthermore, the handle main unit 220 of the handle attachment 216 has an arc shape formed by cutting away a part from a circular shape, and between the arc ends on both sides thereof, the recessed space 35 is provided. Therefore, for example, in the case such as where, with an infusion solution pump being connected to an infusion pack that is hung on the infusion solution hanger, an infusion tube is connected to a patient via the infusion solution pump, it is possible to route tubes, which have been routed from the infusion pack, downwardly via the recessed space 35 without interference with the handle main unit 220. Therefore, it is possible to further enhance the workability and usability at the time of infusion.

Here, description has been for the case where the through-hole 23 is arranged at a position that is offset from the central position of the tray 21. However, as is the case with a modification shown in FIG. 9B, the through-hole 23 may be arranged at the central position of the tray 21.

### (Fourth embodiment)

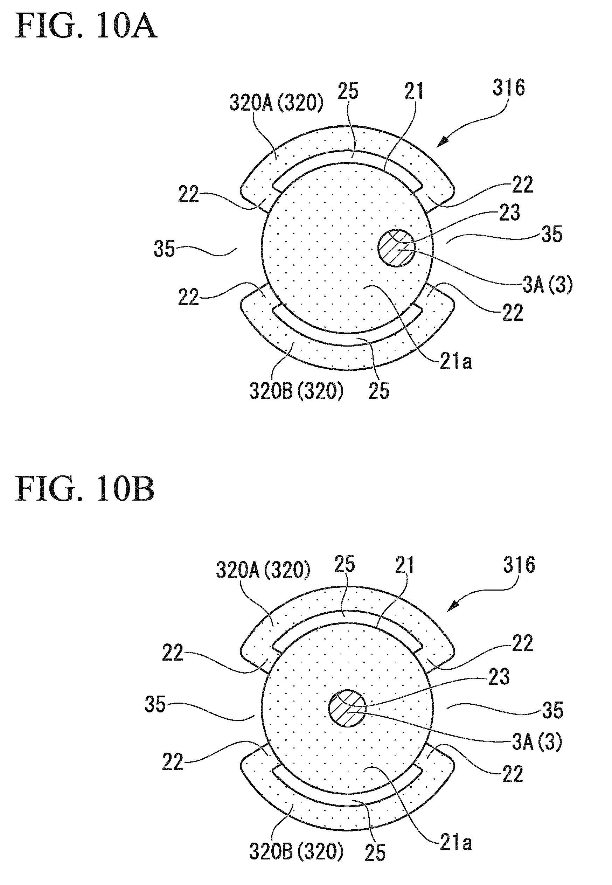

FIG. 10A is a top view of a portion of a handle attachment 316 according to a fourth embodiment.

The handle attachment 316 of this embodiment includes: a disc-shaped tray 21 whose upper surface is an object placing surface 21a; and a handle main unit 320 (a guide member) that is arranged above the tray 21 so as to be spaced a predetermined distance away from the tray 21. Moreover, a holding space 25 is secured between the handle main unit 320 and the tray 21. The handle main unit 320 is made of a pair of arc-like handle pieces 320A, 320B. Both ends of each of the handle pieces 320A, 320B are coupled to an outer circumferential edge of the tray 21 by coupling poles 22. In this embodiment, between adjacent portions of the handle pieces 320A, 320B, there are provided recessed spaces 35. In the tray 21, there is provided a through-hole 23, through which a support pole unit 3 is inserted in the vertical direction, at a position that is offset to one of the recessed spaces 35 with respect to the central position of the tray 21.

With the IV stand of this embodiment, it is possible to obtain an advantageous effect basically similar to that of the third embodiment. Furthermore, two recessed spaces 35, 35 are provided at opposite positions on the circumference of the handle main unit 320. This makes it possible to route tubes downwardly at a plurality of locations around the tray 21 at the time of infusion operations or on other occasions. Therefore, it is possible to further enhance the workability and usability at the time of infusion.

Note that, also in this embodiment, the through-hole 23 may be arranged at the central position of the tray 21 as is the case with a modification shown in FIG. 10B.

### (Fifth embodiment)

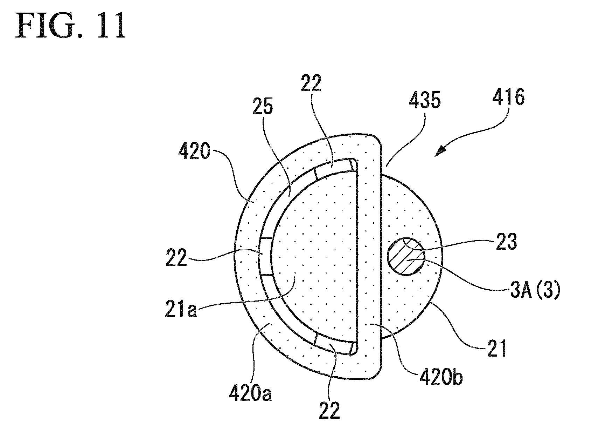

FIG. 11 is a top view of a portion of a handle attachment 416 according to a fifth embodiment.

The handle attachment 416 of this embodiment includes: a disc-shaped tray 21 whose upper surface is an object placing surface 21a; and a handle main unit 420 (a guide member) that is arranged above the tray 21 so as to be spaced a predetermined distance away from the tray 21. Moreover, a holding space 25 is secured between the handle main unit 420 and the tray 21. In the tray 21, there is provided a through-hole 23, through which a support pole unit 3 is inserted, at a position that is offset from the central position of the tray 21. The handle main unit 420 is formed in a substantially D-shape that includes: a semicircular arc portion 420a; and a linear section 420b that linearly couples both ends of the arc portion 420a to each other. The handle main unit 420 is arranged at a position that is offset outwardly from a support pole unit 3 above the upper side of the tray 21. The arc portion 420a of the handle main unit 420 is formed so as to have an outer diameter a size larger than that of the tray 21, and is coupled to an outer circumferential edge of the tray 21 by three coupling poles 22 so as to be coaxial with the center of the tray 21. Furthermore, the linear section 420b of the handle main unit 420 is arranged so as to extend across the area above the tray 21 along a position that is offset toward the outside of the support pole unit 3. In this embodiment, a substantially semicircular space 435 is provided on the outside of the linear section 420b of the handle main unit 420.

With an IV stand of this embodiment, it is possible to obtain basically similar advantageous effect to that of the third and fourth embodiments. Furthermore, the handle main unit 420 is formed in a substantially D-shape, the support pole unit 3 is arranged on the outside of the handle main unit 420, and the space 435 with a substantially semicircular D-shape is provided on the outside of the handle main unit 420. Therefore, it is possible to secure larger space for downwardly routing tubes at a position adjacent to the outside of the handle main unit 420. Moreover, it is possible to prevent an object placed on the object placing surface 21a of the tray 21 from interfering with the support pole unit 3.

### (Sixth embodiment)

Hereunder is a description of embodiments of this invention with reference to the drawings.

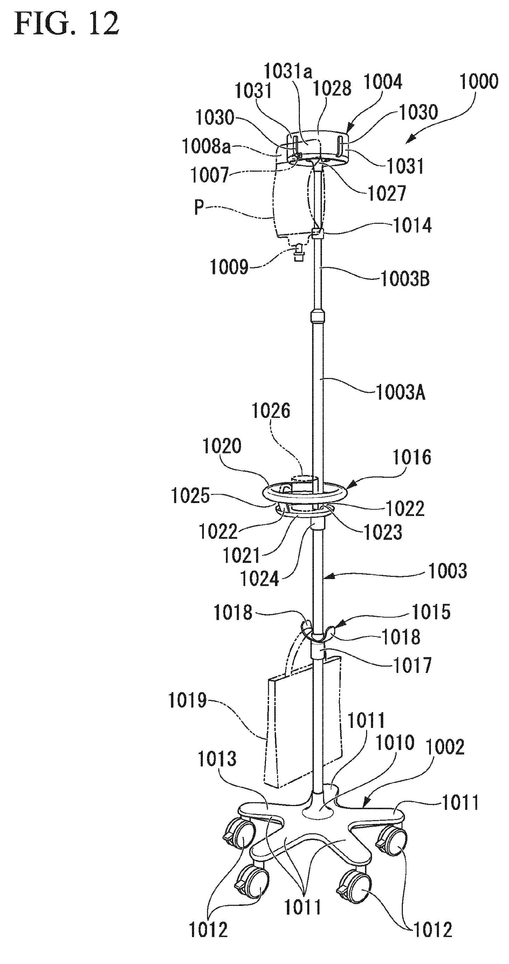

Firstly, a sixth embodiment shown in FIG. 12 to FIG. 17 will be described. In the embodiments and modifications described below, the same parts are denoted with the same reference symbols, and will not be repetitiously explained.

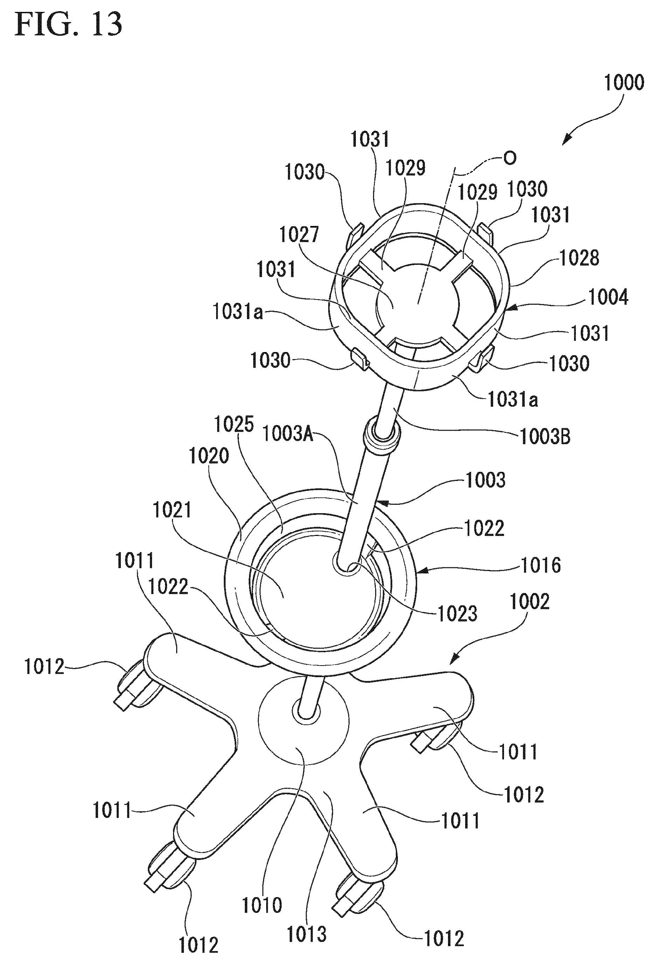

FIG. 12 and FIG. 13 are perspective views showing external appearances of an IV stand 1001 according to this embodiment.

The IV stand 1001 includes: a leg unit block 1002 that is placed on a floor surface of a hospital, a nursing home, a home of a patient, or the like; a support pole unit 1003 that is supported on the leg unit block 1002 and extends in the vertical direction; and an infusion solution hanger 1004 that is attached to an upper end of the support pole unit 1003 and hangingly supports an infusion pack P.

The leg unit block 1002 includes: five leg frames 1011 extending radially toward an outer circumferential side of a boss section 1010 which sustains a lower end of the support pole unit 1003; and casters 1012, each of which is attached to an extension end of each leg frame 11. Therefore, the leg unit block 1002 of this embodiment is movable on the floor surface via the casters 1012. In the case of this embodiment, an upper side of the boss section 1010 and the leg frames 1011 is actually covered with a cover member 1013 made of resin. However, in FIG. 12 and FIG. 13, the sections on the cover member 1013 that correspond to the boss section 1010 and the leg frames 1011 are denoted with reference symbols 1010, 1011, for convenience of illustration.

In the case of this embodiment, the support pole unit 1003 includes: a hollow outer pole 1003A having a lower end which is attached to the leg unit block 1002; and a hollow inner pole 1003B having an upper end to which the infusion solution hanger 1004 is attached. The lower section of the inner pole 1003B is slidably inserted into the upper portion of the outer pole 1003A. The inside of the support pole unit 1003 is equipped with a lock mechanism (not shown in the figures) for fixing the relative positional relationship between the outer pole 1003A and the inner pole 1003B. With the lock mechanism released, it is possible to appropriately adjust the relative positional relationship between the outer pole 1003A and the inner pole 1003B (a telescopic length of the support pole unit 1003).

In a substantially middle position of the inner pole 1003B in the vertical direction, there is installed a release operation sleeve 1014 that is used for releasing the lock mechanism. Inside the inner pole 1003B, the release operation sleeve 1014 is connected to a lock release piece of the lock mechanism. When slid upwardly by an operator such as a nurse, the release operation sleeve 1014 releases the lock mechanism. When slid back downwardly, the release operation sleeve 1014 maintains the lock mechanism in the locked state again.

Furthermore, in this embodiment, to the outer pole 1003A, a hanger hook attachment 1015 and a handle attachment 1016, which is an attachment for the IV stand according to this embodiment, are attached so as to be capable of being risen and lowered in an adjustable manner.

The hanger hook attachment 1015 includes: a boss section 1017 capable of being fastened and fixed to an outer circumferential surface of the outer pole 1003A; and a pair of hook units 1018, 1018 that extend from the boss section 1017 in opposite directions. It is possible to appropriately hang an urobag, belongings 1019, or the like on each of the hook units 1018. The handle attachment 1016 will be described in detail later.

The infusion solution hanger 1004 includes: a boss section 1027 that is fastened and fixed to an upper end of the inner pole 1003B of the support pole unit 1003; a circumferential wall 1028 with a substantially square cylindrical shape that is erected along the vertical direction so as to surround an axis o of the support pole unit 1003; coupling poles 1029 that couple the boss section 1027 to the circumferential wall 1028; and four hanger support pieces 1030 that protrude from an outside surface of a lower end of the circumferential wall 1028 to the outside (the side opposite to the side on which the axis o of the support pole unit 1003 is located).

The circumferential wall 1028 is formed of four support walls 1031 with both ends thereof in the left-right direction curved in an arc shape, in which the support walls 1031 are arranged in a square shape so that adjacent support walls 1031, 1031 are coupled to each other. In the case of this embodiment, the whole of the circumferential wall 1028 is integrally formed from a rigid resin material into a square shape. An outer surface 1031a of each support wall 1031 (a surface on the side opposite to the side on which the axis o of the support pole unit 1003 is located) is formed in a substantially plain shape centering around the central region thereof in the width direction.

The number of the coupling poles 1029 is four, each of which is provided so as to couple a lower edge of the corresponding support wall 1031 at a central position in the width direction to the outer surface of the boss section 1027. The hanger support piece 1030 is made of a metal plate member that is formed in a substantially L-shape in a cross-sectional view. A first edge of the L-shape is attached to the lower edge of the corresponding support wall 1031 at a central position in the width direction while a second edge of the L-shape extends in the upward direction. A front end of the first edge of the L-shape of the hanger support piece 1030 is inserted between the corresponding support wall 1031 and the corresponding coupling pole 1029, and is fixed to the coupling pole 1029. With each hanger support piece 1030, a hanging hole 1007 of the infusion pack P is engageable, as shown in FIG. 12. Reference symbol 1008a in FIG. 12 denotes an upper sealed section of the infusion pack P in which the hanging hole 1007 is formed, and reference symbol 1009 denotes an outlet pipe of the infusion pack P to which an infusion tube (not shown in the figure) is to be connected.

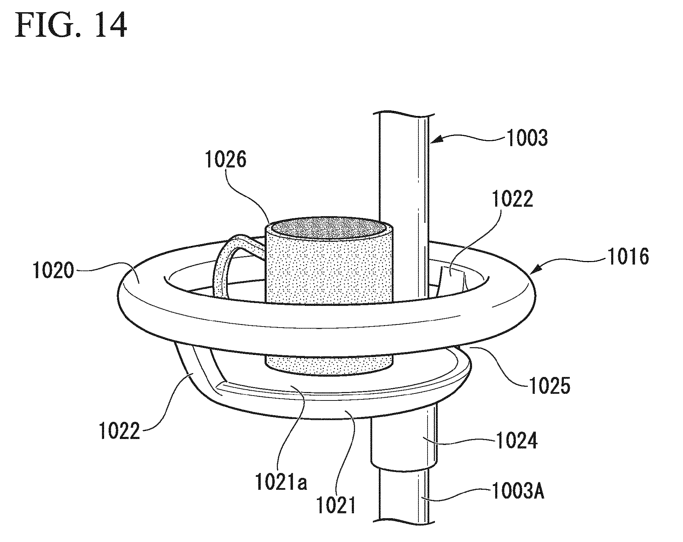

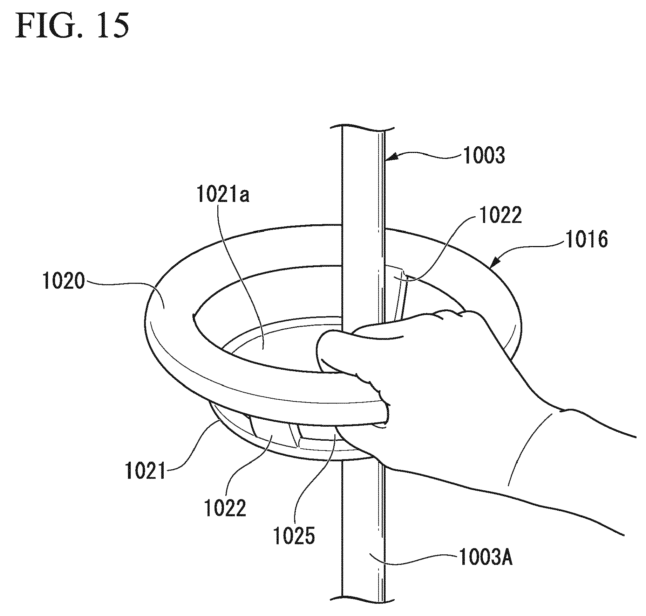

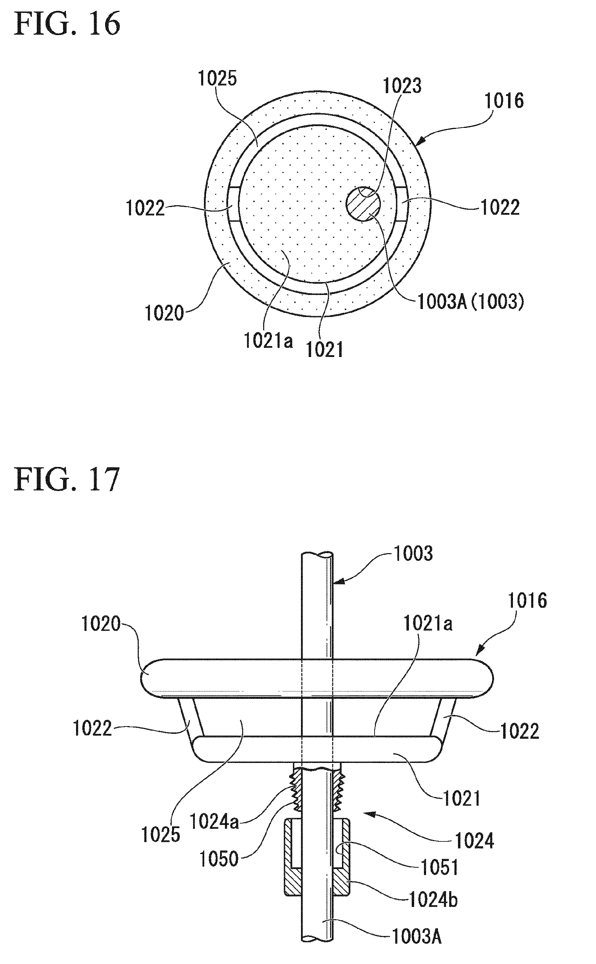

FIG. 14 and FIG. 15 are perspective views showing a portion of the handle attachment 1016. FIG. 16 is a top view of a portion of the handle attachment 1016 shown with the support pole unit 1003 cut away. FIG. 17 is a side view of a portion of the handle attachment 1016, which is shown partially in cross-section.

The handle attachment 1016 includes, as an integral entity,: a tray 1021 having an upper surface on which an object 1026 including an everyday item such as a cup or the like is capable of being placed; a holding handle 1020 (a guide member), which is an annular holding handle arranged above the tray 1021 so as to be spaced a predetermined distance away from the tray 1021; a pair of coupling poles 1022, 1022 (coupling units) that couple the tray 1021 to the holding handle 1020; and a boss section 1024 (a fixing unit) that is fastenable and fixable to an outer circumferential surface of the outer pole 1003A.

The tray 1021 is formed in a circular flat plate that is a size smaller than an inner circumferential surface of the holding handle 1020. An upper surface of the tray 1021 is an object placing surface 1021a for placing the object 1026. The tray 1021 is arranged below the holding handle 1020 so as to be coaxial with the holding handle 1020, and is coupled to the holding handle 1020 by the coupling poles 1022, 1022 at positions opposite to each other on the outer circumference of the tray 1021. The coupling poles 1022 are arranged in an inclined manner so as to couple the upper surface of the tray 1021 on the outer circumferential side to the lower surface of the holding handle 1020 on the inner circumferential side. The tray 1021 is provided with a through-hole 1023 that penetrates vertically therethrough at a position that is offset from its central portion. Through the through-hole 1023, the outer pole 1003A of the support pole unit 1003 is inserted.

In the case of this embodiment, the tray 1021, the coupling poles 1022, 1022, and the holding handle 1020 are formed from a rigid resin so as to be integral with a part of the boss section 1024.

Furthermore, the boss section 1024 is provided at a position so as to be coaxial with the through-hole 1023 on the lower surface of the tray 1021. The boss section 1024 is made of: an inner cylinder 1024a, with a cylindrical shape divided into half, that is provided so as to protrude from the lower surface of the tray 1021; and an outer cylinder 1024b that is fitted onto an outside of the inner cylinder 1024a. The inner cylinder 1024a is deformable inwardly in the radial direction about the divided section, and is provided with an external thread 1050 in the outer circumferential surface thereof. The outer cylinder 1024b is provided with an internal thread 1051 that is threadedly engageable with the external thread 1050 of the inner cylinder 1024a. With the internal thread 1051 being screwed onto the external thread 1050 of the inner cylinder 1024a, it is possible to press-fix the inner cylinder 1024a onto the outer circumferential surface of the support pole unit 1003 (the outer pole 1003A). Therefore, with the screwing of the outer cylinder 1024b of the boss section 1024 being released, the handle attachment 1016 is movable on the support pole unit 1003 (the outer pole 1003A). With the outer cylinder 1024b being screwed again at a desired position, it is possible to fix the handle attachment 1016 to the support pole unit 1003 (the outer pole 1003A).

Here, below the holding handle 1020 coupled to the tray 1021 by the pair of coupling poles 1022, 1022, a holding space 1025 is secured that is wide enough for an operator to hold the holding handle 1020. Therefore, unless the caster 1012 of the leg unit block 1002 is locked, an operator such as a patient or a nurse is capable of moving the IV stand 1001 freely in any direction while holding the holding handle 1020.

Furthermore, although the holding space 1025 is secured between the holding handle 1020 and the tray 1021, the height from the upper surface of the tray 1021 to the holding handle 1020 is set to be lower than that of the object 1026 that a patient uses in his or her daily life such as a cup. Therefore, the holding handle 1020 also functions as a guide member (a restriction unit) that restricts the object 1026 placed on the tray 1021 from falling down. In the case of this embodiment, the through-hole 1023 in the tray 1021 through which the outer pole 1003A is inserted is arranged at a position that is offset from the center of the tray 1021. Therefore, on the tray 1021, enough object placement space is secured that does not interfere with the outer pole 1003A.

Furthermore, in this embodiment, the whole area of the upper surface of the holding handle 1020 is formed so as to be substantially horizontal. Therefore, as required, it is also possible to place a large object such as an IV pack P on the upper surface of the holding handle 1020.

As described above, in the IV stand 1001 according to this embodiment, the tray 1021 and the holding handle 1020 are spaced in the vertical direction, to thereby secure a holding space. Therefore, it is possible to make the outer shape of the handle attachment 1016 compact in size as a whole.

Furthermore, in the IV stand 1001 according to this embodiment, the tray 1021 and the holding handle 1020 of the handle attachment 1016 are coupled to each other by the coupling poles 1022 so as to secure the holding space 1025 between the holding handle 1020 and the tray 1021, and the lower surface side of the tray 1021 is provided integrally with the boss section 1024. Therefore, it is possible to integrally combine the placement function portion of the object 1026 and the holding handle function portion in a compact manner and to attach them with ease around the support pole unit 1003. As a result, in the IV stand 1001 of this embodiment, it is possible to achieve both of the placement of the object 1026 around the support pole unit 1003 and the secure holding of the IV stand 1001 by the operator in moving on other occasions, without deteriorating the appearance around the support pole unit 1003 and a decrease in workability for attachment.

Furthermore, especially in the IV stand 1001, the holding handle 1020 of the handle attachment 1016 functions also as a guide member (a restriction unit) that restricts the object 1026 placed on the object placing surface 1021a of the tray 1021 from falling down. Therefore, it is possible to further enhance the usability when the object 1026 is placed on the tray 1021. Therefore, in the case of the IV stand 1001, with the handle attachment 1016 being provided with the function of restricting the object 1026 from falling down, it is possible to limit an increase in space occupied by the handle attachment 1016 and to make the appearance of the whole unit favorable.

Furthermore, there may be a case of arranging the tray 1021 of the handle attachment 1016 outside the support pole unit 1003 by using a coupling arm or the like that extends from a side of the tray 1021. On the other hand, in the handle attachment 1016 of this embodiment, it is possible to decrease the amount of outward extension of the tray 1021 from the support pole unit 1003 because the tray 1021 is provided with the through-hole 1023 and the support pole unit 1003 is inserted through the through-hole 1023. Therefore, in the case of adopting the handle attachment 1016, it is possible to further enhance the usability and appearance of the IV stand 1001.

Furthermore, in the IV stand 1001 of this embodiment, the through-hole 1023 is provided at a position that is offset from the central position of the tray 1021 of the handle attachment 1016 when seen in the top view. Consequently, it is possible to secure, on the tray 1021, adequate space for placing an object that does not interfere with the support pole unit 1003. Therefore, in the IV stand 1, it is possible to inhibit the amount of outward extension of the tray 1021 while maintaining the easiness with which the object 1026 is placed on the tray 1021 of the handle attachment 1016.

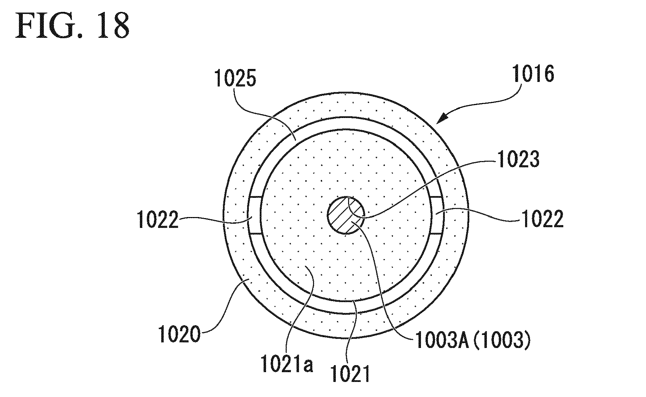

However, in the handle attachment 1016, the through-hole 1023 is not necessarily required to be provided at a position that is offset from the central position of the tray 1021. As is the case with a modification shown in FIG. 18, the through-hole 1023 may be provided at the central position of the tray 1021.

Furthermore, the holding handle 1020 may not necessarily be arranged above the tray 1021 so as to be along the outer circumferential edge of the tray 1021. However, in the handle attachment 1016 of this embodiment, the holding handle 1020 is arranged above the tray 1021 so as to be along the outer circumferential edge of the tray 1021. Therefore, it is possible to effectively utilize a wide area above the tray 1021 as the object placing surface 1021 a.

Especially in the case of this embodiment, the holding handle 1020 of the handle attachment 1016 is arranged so that the inner circumferential surface of the holding handle 1020 is outer than the outer circumferential surface of the tray 1021 when seen in a top view. Therefore, it is possible to effectively utilize substantially the whole area of the upper surface of the tray 1021 as the object placing surface 1021a. Furthermore, this offers an advantage in that, when the operator holds the holding handle 1020, the tray 1021 is unlikely to be an obstacle.

Note that the holding handle 1020 may be formed so that the inner circumferential surface of the holding handle 1020 and the outer circumferential surface of the tray 1021 are in substantially the same position when seen in a top view.

Furthermore, in the IV stand 1001 of this embodiment, the holding handle 1020 that is arranged above the tray 1021 of the handle attachment 1016 is formed so as to have a similar shape to an outer shape of the tray 1021. Therefore, the balance in external appearance is favorable, and the appearance from the outside is not deteriorated.

Furthermore, the holding handle 1020 of the handle attachment 1016 is not necessarily required to have an annular shape. However, in the handle attachment 1016 of this embodiment, the holding handle 1020 is formed in an annular shape. Therefore, when holding the holding handle 1020, the operator is capable of holding the holding handle 1020 from any direction in the circumferential area. This produces an advantage of favorable operability.

### (Seventh embodiment)

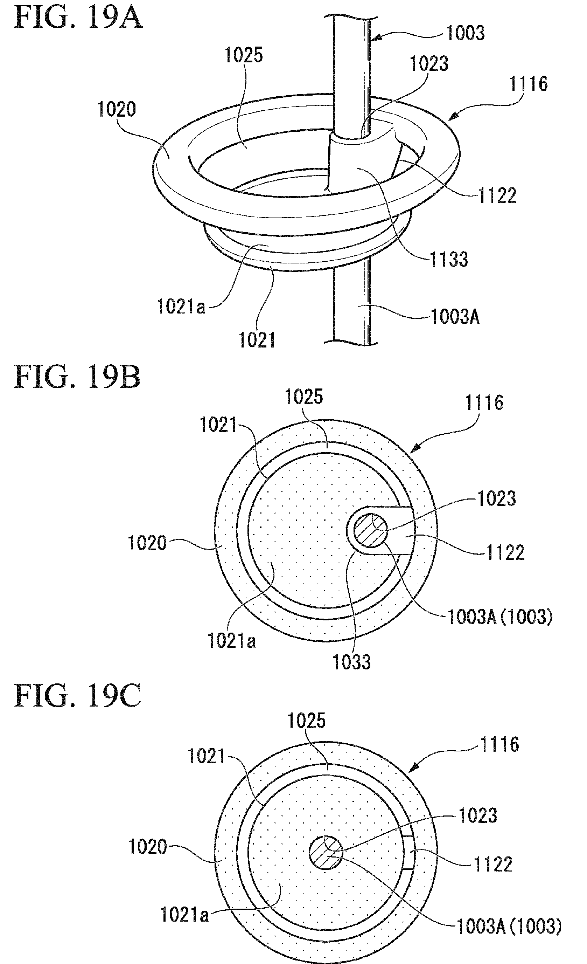

Subsequently, other embodiments shown in FIG. 19A to FIG. 23 will be described. The embodiments shown in FIG. 19A to FIG. 23 are different from the aforementioned sixth embodiment only in the structure of the handle attachment, and otherwise have similar structures.

FIGS. 19A and 19B are a perspective view and a top view of a portion of a handle attachment 1116 of a seventh embodiment.

The handle attachment 1116 of this embodiment includes: a disc-shaped tray 1021 whose upper surface is an object placing surface 1021a; and an annular holding handle 1020 that is arranged above the tray 1021 so as to be spaced a predetermined distance away from the tray 1021. The holding handle 1020 is arranged so as to be coaxial with the tray 1021, and a holding space 1025 is secured between the holding handle 1020 and the tray 1021. Also in the case of this embodiment, the inner circumferential surface of the holding handle 1020 is formed in a circular shape so as to be a size larger than the outer circumferential surface of the tray 1021. In the sixth embodiment, the holding handle 1020 and the tray 1021 are coupled by a pair of coupling poles that are arranged at opposite positions on the circumference. However, in this embodiment, the holding handle 1020 and the tray 1021 are coupled by a single coupling pole 1122.

On the coupling pole 1122, a swelling portion 1033 with a trapezoidal shape is integrally formed that swells inwardly in the radial direction from the inner circumferential surface of the holding handle 1020 to the outer circumferential edge of the tray 1021. In the swelling portion 1033 and in a site of the tray 1021 that is coupled to the swelling portion 1033, there is formed a through-hole 1023 through which the outer pole 1003A of the support pole unit 1003 is inserted in the vertical direction. The swelling portion 1033 constitutes at least a part of a fixing unit which fixes the handle attachment 1116 to the support pole unit 1003. In the case of this embodiment, the through-hole 1023 is arranged at a position that is offset from a central position of the tray 1021. Note that a fixation device for press-fixing the handle attachment 1116 to the support pole unit 1003 may be provided in the swelling portion 1033, or may be provided on the lower surface side of the tray 1021 similarly to the case of the sixth embodiment.

With the handle attachment 1116 of this embodiment, it is possible to obtain a basic advantageous effect similar to that of the sixth embodiment. Furthermore, the holding handle 1020 is coupled by the coupling pole 1122 only at a single location in the circumferential direction. Therefore, when an operator holds the holding handle 1020, it is possible to expand the area in which the operator can hold the holding handle 1020 without interfering with the coupling pole 1122, to thereby enhance the operability. Furthermore, it is possible to further enhance the quality in external appearance.

Furthermore, in the case of this embodiment, the coupling pole 1122 that couples the holding handle 1020 to the tray 1021 is provided with the swelling portion 1033 constituting at least a part of the fixing unit. Therefore, it is possible to make the handle attachment 1116 itself compact in size.

Here, description has been for the case where the through-hole 1023 is arranged at a position that is offset from the central position of the tray 1021. However, as is the case with a modification shown in FIG. 19C, the through-hole 1023 may be arranged at the central position of the tray 1021. In the case of this modification, the coupling pole 1122 is not provided with a swelling portion, and the tray 1021 and the holding handle 1020 are coupled by the coupling pole 1122 in a slanting manner.

### (Eighth embodiment)

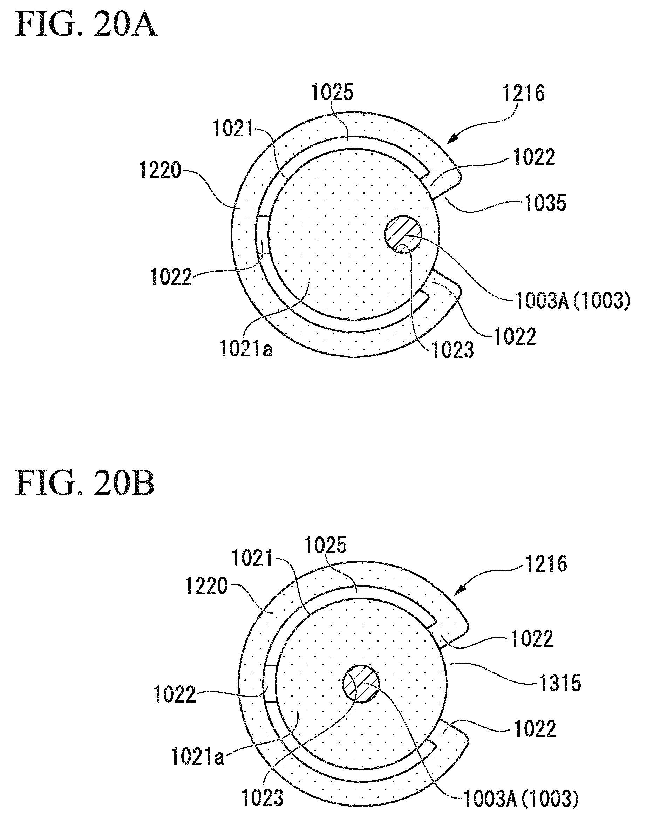

FIG. 20A is a top view of a portion of a handle attachment 1216 according to an eighth embodiment.

The handle attachment 1216 of this embodiment includes: a disc-shaped tray 1021 whose upper surface is an object placing surface 1021a; and a holding handle 1220 (a guide member) that is arranged above the tray 1021 so as to be spaced a predetermined distance away from the tray 1021. Moreover, a holding space 1025 is secured between the holding handle 1220 and the tray 1021. However, in the case of this embodiment, the holding handle 1220 does not have a perfect annular shape, but has an arc shape formed by cutting away a part from a circular shape. The holding handle 1220 is arranged so as to be coaxial with the tray 1021. The handle main unit 1220 is coupled to an outer circumferential edge of the tray 1021 by three coupling poles 1022 at arc ends on both sides of the handle main unit 1220 and at a central portion of the arc. Furthermore, at a position that is offset from the central position of the tray 1021, there is provided a through-hole 1023 through which a support pole unit 1003 is inserted. The inner surface of the holding handle 1220 is formed in a circular shape that is a size larger than the outer circumferential surface of the tray 1021.

Furthermore, between the arc ends on both sides of the holding handle 1220, there is provided a recessed space 1035 that is recessed in a substantially delta-shape toward the outer circumferential surface of the tray 1021 when seen in a top view.

With the handle attachment 1216 of this embodiment, it is possible to obtain a basic advantageous effect similar to that of the sixth embodiment. Furthermore, the holding handle 1220 has an arc shape formed by cutting away a part from a circular shape, and between the arc ends on both sides thereof, the recessed space 1035 is provided. Therefore, for example, in the case such as where, with an infusion solution pump being connected to an infusion pack that is hung on the infusion solution hanger, an infusion tube is connected to a patient via the infusion solution pump, it is possible to route tubes, which have been routed from the infusion pack, downwardly via the recessed space 1035 without interference with the holding handle 1220. Therefore, it is possible to further enhance the workability and usability at the time of infusion.

Here, description has been for the case where the through-hole 1023 is arranged at a position that is offset from the central position of the tray 1021. However, as is the case with a modification shown in FIG. 20B, the through-hole 1023 may be arranged at the central position of the tray 1021.

### (Ninth embodiment)

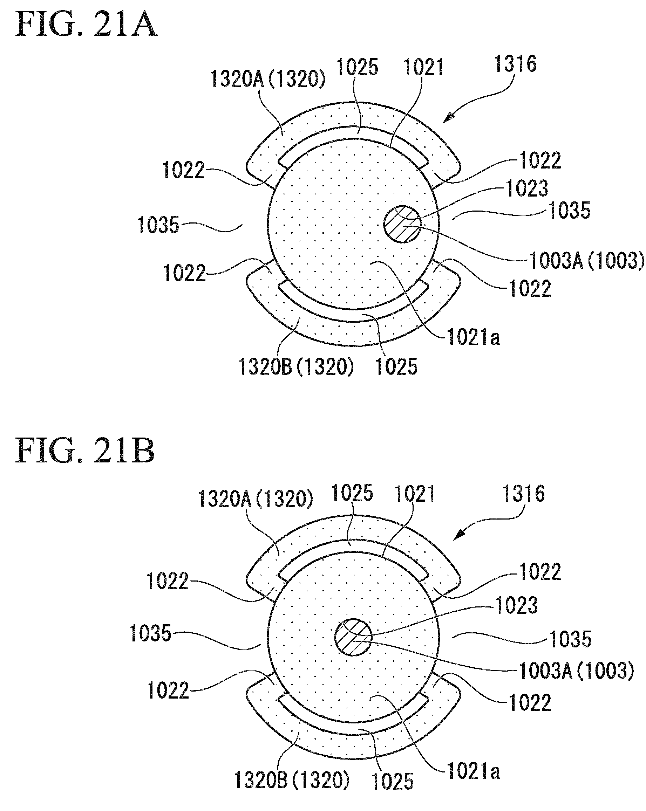

FIG. 21A is a top view of a portion of a handle attachment 1316 according to a ninth embodiment.

The handle attachment 1316 of this embodiment includes: a disc-shaped tray 1021 whose upper surface is an object placing surface 1021a; and a holding handle 1320 (a guide member) that is arranged above the tray 1021 so as to be spaced a predetermined distance away from the tray 1021. Moreover, a holding space 1025 is secured between the holding handle 1320 and the tray 1021. The holding handle 1320 is made of a pair of arc-like handle pieces 1320A, 1320B. Both ends of each of the handle pieces 1320A, 1320B are coupled to an outer circumferential edge of the tray 1021 by coupling poles 1022. In this embodiment, between adjacent portions of the handle pieces 1320A, 1320B, there are provided recessed spaces 1035. In the tray 1021, there is provided a through-hole 1023, through which a support pole unit 1003 is inserted in the vertical direction, at a position that is offset to one of the recessed spaces 1035 with respect to the central position of the tray 1021.

With the handle attachment 1316 of this embodiment, it is possible to obtain an advantageous effect basically similar to that of the eighth embodiment. Furthermore, two recessed spaces 1035, 1035 are provided at opposite positions on the circumference of the holding handle 1320. This makes it possible to route tubes downwardly at a plurality of locations around the tray 1021 at the time of infusion operations or on other occasions. Therefore, it is possible to further enhance the workability and usability at the time of infusion.

Note that, also in this embodiment, the through-hole 1023 may be arranged at the central position of the tray 1021 as is the case with a modification shown in FIG. 21B.

### (Tenth embodiment)

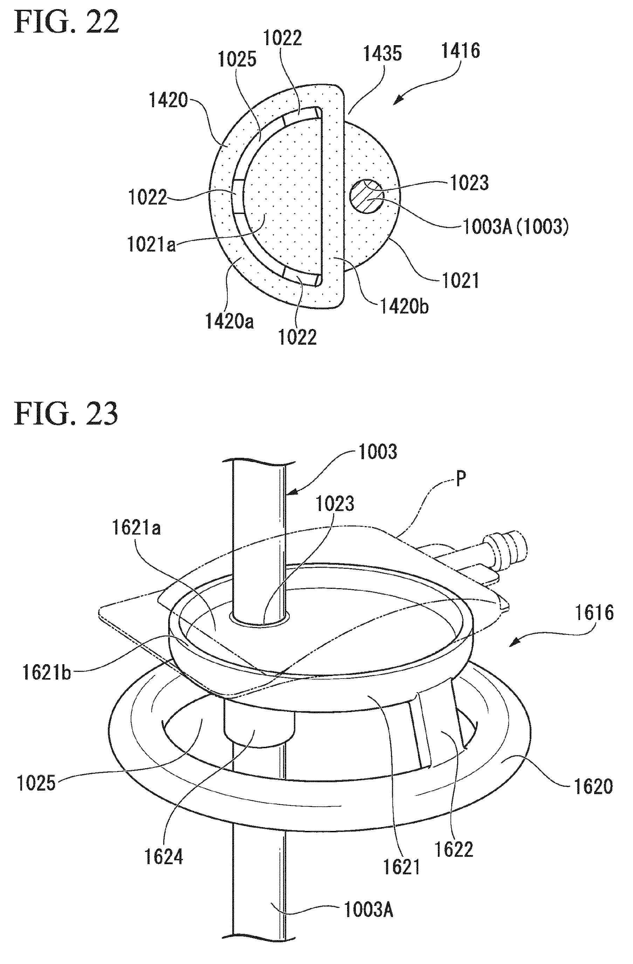

FIG. 22 is a top view of a portion of a handle attachment 1416 according to a tenth embodiment.

The handle attachment 1416 of this embodiment includes: a disc-shaped tray 1021 whose upper surface is an object placing surface 1021a; and a holding handle 1420 (a guide member) that is arranged above the tray 1021 so as to be spaced a predetermined distance away from the tray 1021. Moreover, a holding space 1025 is secured between the holding handle 1420 and the tray 1021. In the tray 1021, there is provided a through-hole 1023, through which a support pole unit 1003 is inserted, at a position that is offset from the central position of the tray 1021. The holding handle 1420 is formed in a substantially D-shape that includes: a semicircular arc portion 1420a; and a linear section 1420b that linearly couples both ends of the arc portion 1420a to each other. The holding handle 1420 is arranged at a position that is offset outwardly from a support pole unit 1003 above the upper side of the tray 1021. The arc portion 1420a of the holding handle 1420 is formed so as to have an outer diameter a size larger than that of the tray 1021, and is coupled to an outer circumferential edge of the tray 1021 by three coupling poles 1022 so as to be coaxial with the center of the tray 1021. Furthermore, the linear section 1420b of the holding handle 1420 is arranged so as to extend across the area above the tray 1021 along a position that is offset toward the outside of the support pole unit 1003. In this embodiment, a substantially semicircular space 1435 is provided on the outside of the linear section 1420b of the holding handle 1420.

With the handle attachment 1416 of this embodiment, it is possible to obtain basically similar advantageous effect to that of the eighth and ninth embodiments. Furthermore, the holding handle 1420 is formed in a substantially D-shape, the support pole unit 1003 is arranged on the outside of the holding handle 1420, and the space 1435 with a substantially semicircular D-shape is provided on the outside of the holding handle 1420. Therefore, it is possible to secure larger space for downwardly routing tubes at a position adjacent to the outside of the holding handle 1420. Moreover, it is possible to prevent an object placed on the object placing surface 1021a of the tray 1021 from interfering with the support pole unit 1003.

### (Eleventh embodiment)

FIG. 23 is a perspective view of a portion of a handle attachment 1616 according to an eleventh embodiment.

The handle attachment 1616 of this embodiment includes: a disc-shaped tray 1621 whose upper surface is capable of placing an object; and an annular holding handle 1620 that is arranged below the tray 1621 so as to be spaced a predetermined distance away from the tray 1621. Moreover, a holding space 1025 is secured between the holding handle 1620 and the tray 1621. The holding handle 1620 is arranged so as to be coaxial with the tray 1621, and is coupled to an outer circumferential edge of the tray 1621 by a pair of coupling poles 1622 (coupling units) that are arranged at opposite positions on the circumference of the tray 1621. In the case of this embodiment, the inner circumferential surface of the holding handle 1620 is formed so as to be larger than the outer circumferential surface of the tray 1621.

Furthermore, the tray 1621 is provided with a through-hole 1023, through which a support pole unit 1003 is inserted, at a position that is offset from the central position, and is also provided with a boss section 1624, which is used for attaching the handle attachment 1616 to the support pole unit 1003, on a lower surface of the tray 1621.

In the case of this embodiment, on the upper surface side of the tray 1621, there is provided an annular edge wall section 1621b on an outer circumferential side, and there is provided an object placing surface 1621a with a recessed shape inside the edge wall section 1621b. Furthermore, a top surface of the edge wall section 1621b is formed so as to be substantially horizontal.

In the handle attachment 1616 of this embodiment, the holding space 1025 is provided between the holding handle 1620 and the tray 1621 arranged thereabove. Therefore, in moving the IV stand or on other occasions, the operator is capable of holding the portion of the holding handle 1620. Furthermore, it is possible to place an object such as a cup onto the object placing surface of the tray 1621. Furthermore, because the top surface of the edge wall section 1621b of the tray 1621 is formed so as to be substantially horizontal, it is possible to place a large object such as an infusion pack P on the top surface of the edge wall section 1621b.

Also in the case of this embodiment, the tray 1621 and the holding handle 1620 are coupled by the coupling poles 1622, and the lower surface of the tray 1621 is provided integrally with the boss section 1624 that is used for attaching the tray 1621 to the support pole unit 1003. Therefore, it is possible to combine the placement function portion for the object and the holding handle function portion in a compact size, and to attach the combined portions to the support pole unit 1003 with ease.

Also in the case of this embodiment, the through-hole 1023 may be provided at the central position of the tray 1621.

The present invention is not limited to the aforementioned embodiments, and various changes in design can be made without departing from the spirit or scope of the present invention. For example, in the aforementioned embodiments, the portion of the tray of the handle attachment is attached to the support pole unit. However, instead of the portion of the tray, the portion of the handle main unit may be attached to the support pole unit, or both of the portion of the tray and the portion of the handle main unit may be attached to the support pole unit.

### Industrial Applicability

According to the present invention, it is possible to provide an IV stand that is capable of enhancing the usability and operability without a deterioration in appearance and an increase in space occupied by attachments.

Furthermore, according to the present invention, it is possible to provide an IV stand and an attachment for an IV stand that are capable of enhancing the usability and operability without deteriorating appearance and causing difficulties with attachment.

### Reference Signs List

1, 1001: IV stand
3, 1003 support pole unit
4, 1004 infusion solution hanger
16, 116, 216, 316, 416, 1016, 1116, 1216, 1316, 1416, 1616: handle attachment (attachment)
20, 220, 320, 420: handle main unit (guide member)
21, 1021, 1621: tray
21a, 1021a, 1621a: object placing surface
22, 122, 1022, 1122, 1622: coupling pole (coupling unit)
23, 1023: through-hole
24, 1024: boss section (fixing unit)
25, 1025: holding space
1020, 1220, 1320, 1420, 1620: holding handle
P: IV pack

## Claims

1. An IV stand in which an infusion pack is hung on a support pole unit, which extends in a vertical direction, via an infusion solution hanger, the IV stand comprising:
a tray having an object placing surface on which an object is capable of being placed; and
a guide member that is arranged above at least a part of the object placing surface of the tray and prevents the object placed on the tray from falling down,
wherein the tray and the guide member are directly or indirectly coupled to the support pole unit, and
a holding space that allows an operator to hold the guide member as a holding handle is secured between the guide member and the tray.

2. The IV stand according to claim 1, wherein the tray is provided with a through-hole through which the support pole unit is inserted in the vertical direction.

3. The IV stand according to claim 2, wherein the through-hole is provided at a position that is offset from a center of the tray when seen in a top view.

4. The IV stand according to claim 1, wherein the guide member is disposed above the tray so as to be along an outer circumferential edge of the tray.

5. The IV stand according to claim 4, wherein an inner circumferential surface of the guide member is formed at a position substantially the same as or further outward than an outer circumferential surface of the tray when seen in a top view.

6. The IV stand according to claim 1, wherein the guide member is formed in a similar shape to an outer edge of the tray when seen in a top view.

7. The IV stand according to claim 1, wherein the guide member is formed in an annular shape when seen in a top view.

8. An IV stand in which an infusion pack is hung on a support pole unit, which extends in a vertical direction, via an infusion solution hanger, the IV stand comprising
an attachment which includes:
a tray having an object placing surface on which an object is capable of being placed;
a holding handle that is spaced away from the tray so as to secure a holding space that allows an operator to hold the holding handle;
a coupling unit that couples the tray to the holding handle; and
a fixing unit that fixes at least one of the tray and the holding handle to the support pole unit.

9. The IV stand according to claim 1, wherein the fixing unit is provided in the coupling unit.

10. The IV stand according to claim 8, wherein the tray is provided with a through-hole through which the support pole unit is inserted in the vertical direction.

11. The IV stand according to claim 8, wherein the holding handle is formed in a similar shape to an outer edge of the tray when seen in a top view.

12. The IV stand according to claim 8, wherein the holding handle is formed in an annular shape when seen in a top view.

13. An attachment for an IV stand that is to be attached to a support pole unit extending in a vertical direction, comprising:
a tray having an object placing surface on which an object is capable of being placed;
a holding handle that is spaced away from the tray so as to secure a holding space that allows an operator to hold the holding handle;
a coupling unit that couples the tray to the holding handle; and
a fixing unit that fixes at least one of the tray and the holding handle to the support pole unit.
